(19) Europäisches Patentamt
European Patent Office
Office européen des brevets

(11) **EP 3 378 404 A1**

(12) **EUROPEAN PATENT APPLICATION**

(43) Date of publication:
26.09.2018 Bulletin 2018/39

(51) Int Cl.:
*A61B 8/06* (2006.01)          *A61B 8/08* (2006.01)

(21) Application number: 17161867.1

(22) Date of filing: 20.03.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Koninklijke Philips N.V.
5656 AE Eindhoven (NL)

(72) Inventors:
• GU, Xiaolin
5656 AE Eindhoven (NL)

• DENG, Yinhui
5656 AE Eindhoven (NL)
• LI, Xiaomin
5656 AE Eindhoven (NL)
• SHAMDASANI, Vijay Thakur
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) **ULTRASONIC DIAGNOSTIC SYSTEM AND METHOD FOR CONTRAST ENHANCED LIVER DIAGNOSIS**

(57) An ultrasonic diagnostic imaging system and method acquire a sequence of image data as a bolus of contrast agent washes into and out of the liver. The image data of contrast intensity is used to compute time-intensity curves of contrast flow in the hepatic artery, the portal vein, and at one or more regions of interest in the liver. The time-intensity curve of contrast flow in the hepatic artery is scaled to the time-intensity curve of contrast flow in a region of interest to produce time-intensity curve data representing the flow of blood at the region of interest due to arterial flow. The time-intensity curve data representing the flow of blood at the region of interest due to arterial flow is subtracted from the time-intensity curve of contrast flow in the region of interest to produce time-intensity curve data representing the flow of blood at the region of interest due to venous flow.

Fig. 8

EP 3 378 404 A1

**Description**

This invention relates to medical diagnostic ultrasound systems and, in particular, to ultrasound systems which perform contrast-enhanced imaging studies to identify and characterize lesions such as liver tumors.

Hepatitis B and hepatitis C patients have been found to be at an increased risk of developing primary liver cancer, hepatocellular carcinoma (HCC). Due to the discovery that hepatitis C was being contracted by patients through blood transfusions in the early 1980's, there remain a significant number of hepatitis C patients who need to be examined regularly for the onset of HCC, as the lesions are best treated in their early stages. The usual progression of the disease is from hepatitis to liver cirrhosis to HCC. An easy-to-use monitoring technique for liver disease progression would have widespread application in assisting in the early detection of this serious disease.

There are a number of lesions in addition to HCC which can develop in the liver. The paper "Ultrasound Contrast Imaging Research" by M. Averkiou et al., published in Ultrasound Quarterly, vol. 19, no. 1 at pp 27 et seq. (2003) discusses four primary liver lesions: hepatocellular carcinoma (HCC), metastasis from a primary tumor at some other location, hemangioma, and focal nodal hyperplasia. The former two are malignant and the latter two are benign. A technique for identifying lesions in the liver should be able to detect all four of these lesions so that more detailed diagnosis can identify and distinguish between all lesions found in the liver.

Since liver lesions, like other cancers, are most effectively treated when detected early, high-risk patients should be monitored frequently for signs of these diseases. But in their early stages liver lesions are often difficult to detect through conventional diagnostic imaging due to their small size. Thus, clinicians often conduct their diagnoses to look for other signs that a lesion is developing. One of these signs is changes in the blood flow to the liver. The liver has a unique blood supply network. A primary source of fresh blood to the liver is the arterial inflow from the hepatic artery. But the liver has a secondary blood supply, the portal vein in the abdomen. Being both arterial and venous, these sources of supply function differently. The pulsatile flow of blood from the hepatic artery occurs shortly after systole, like other arterial vessels. The inflow of blood from the portal vein occurs later in the heart cycle. The relative timing and amount of blood flow from these two sources has been related to the onset of lesions in the liver. Thus, it is desirable to be able to analyze the characteristics of blood flow in the liver from these two sources.

However, the flow of blood in the liver parenchyma is a combination of blood flows from these two sources. It is not possible to discern separately the amount of blood arriving in regions of interest in the liver from these separate sources. It is desirable to be able to separate the flow of blood in different regions of interest in the liver as it relates to the hepatic arterial and portal venous flows, so that inferences about lesion development can be accurately drawn.

In accordance with the principles of the present invention a system and method are described for identifying the sources of blood flow in regions of interest in the liver as being arterial or venous. The present inventors have noted that the combined blood flow in the liver is linearly related to inflows from both the hepatic artery and the portal vein. Time-intensity curves (TICs) of blood flow in the hepatic artery and portal vein are acquired from the blood flow in these vessels where they enter the liver and are used as references for these separate sources of blood supply. A time-intensity curve is also acquired of the perfusion of blood in a region of interest (ROI) in the liver parenchyma. The initial portion of the TIC of the ROI, when inflow is only from the hepatic artery, is related to the hepatic artery time-intensity curve. A scaled replica of the hepatic artery TIC is subtracted from the TIC of the ROI to separate the TIC into two curves, one relating to hepatic arterial inflow and the other relating to portal vein inflow. A display is then produced of one or more characteristics of the time-intensity curves of both sources of blood flow in the liver.

In the drawings:

FIGURE 1 is an illustration of the liver, showing the entry of the hepatic artery and portal vein into the bottom of the organ.

FIGURE 2 illustrates contrast and B mode images of a liver showing the location of the hepatic artery entering the liver.

FIGURE 3 illustrates contrast and B mode images of a liver showing the location of the portal vein entering the liver.

FIGURE 4 illustrates time-intensity curves of contrast flow in the hepatic artery and in the portal vein.

FIGURE 5 illustrates a simplified time-intensity curve of contrast flow in the hepatic artery.

FIGURE 6 illustrates a simplified time-intensity curve of contrast flow in the portal vein.

FIGURE 7 illustrates a typical time-intensity curve of perfusion in a region of interest in the liver.

FIGURE 8 illustrates in block diagram form an ultrasonic diagnostic imaging system constructed in accordance with the principles of the present invention.

FIGURE 9 is a flow chart of a method for conducting liver diagnosis in accordance with the principles of the present invention.

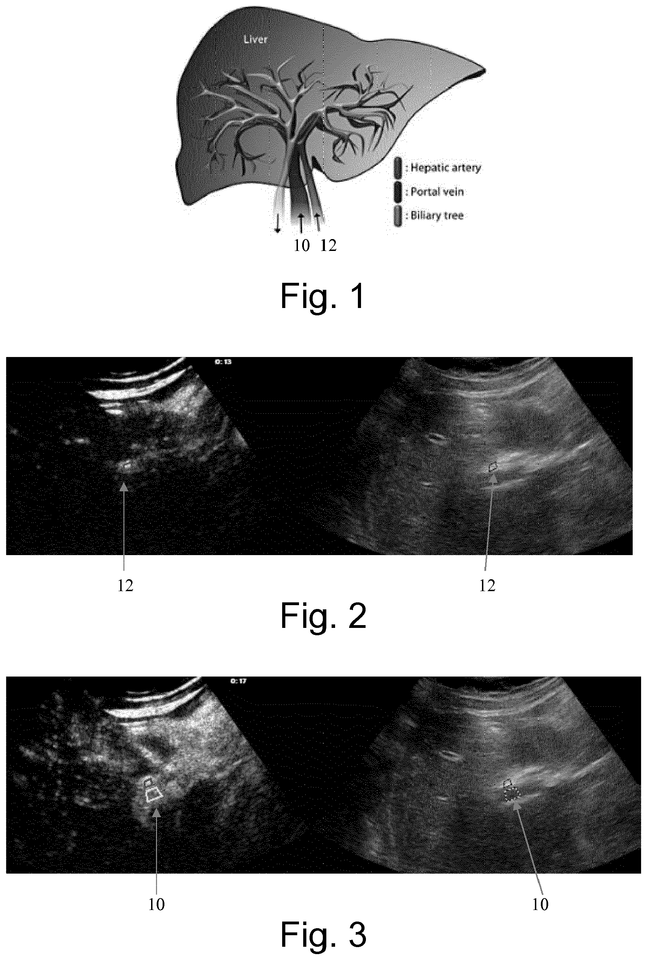

FIGURE 1 is an illustration of the liver and its flow network. The liver contains vessel trees for three sources of flow: the biliary tree for the flow of liver bile, the portal vein 10 which provides a nourishing flow of blood from the abdomen, and the hepatic artery 12 which is a source of arterial blood flow. The capillary structures of these networks are intertwined throughout the parenchyma, causing liver tissue to be perfused with blood from both arterial and venous sources. The hepatic artery 12 and portal vein 10 can be accessed separately where they enter the organ at the bottom of the liver as shown in the illustration.

FIGURE 2 shows two ultrasound images, a contrast image on the left and a B mode image on the right, illustrating the location of the hepatic artery 12 and its blood flow. The two images were acquired at the same time with the vascular system perfused with a microbubble contrast agent. The contrast image on the left was processed by enhancing the harmonic (nonlinear) signal return from microbubbles while suppressing fundamental signal frequencies from tissue, whereas the B mode image on the right was produced from fundamental frequency signals with harmonic frequencies suppressed.

The hepatic artery 12 is outlined in both images. The hepatic artery 12 is easier to locate in the left contrast image due to its cyclical brightening with each heartbeat as contrast perfused blood is pumped through the vessel. Showing both the contrast and tissue images at the same time assists the sonographer in stabilizing the probe during image acquisition. The right tissue image, in which contrast flow is suppressed, should be relatively static and helps the sonographer maintain the probe constantly aligned with the same scan plane, while the pulsatile flow in the more dynamic contrast image assists in spotting the hepatic artery from its cyclical blood flow.

FIGURE 3 is another pair of matching contrast and tissue images, this time with a tracing of the portal vein 10 added to the images. Consistent with the illustration of FIGURE 1, it is seen that the hepatic artery 12 and portal vein 10 can be accessed for scanning adjacent to each other below the liver.

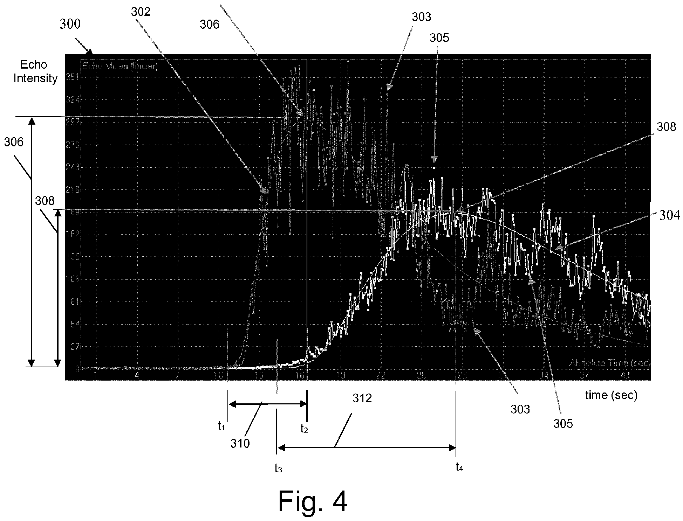

FIGURE 4 is a graph 300 showing first and second time-intensity curves 302 and 304 produced from echo signals acquired over time from contrast-perfused blood flow in the hepatic artery 12 and the portal vein 10, respectively. First contrast intensity information 303 (shown comprising connected points) was produced from harmonic signal returns acquired over time from the hepatic artery 12, and second contrast intensity information 305 was produced from harmonic signal returns acquired over time from the portal vein 10. The first and second contrast information values are processed by the ultrasound system, *e.g.,* by averaging, curve fitting, *etc.,* using any suitable algorithm to respectively form the first and second time-intensity curves 302 and 304, respectively. Accordingly, the first time-intensity curve 302 corresponds with (*e.g.,* by fitting, averaging, *etc.*) the first contrast intensity information 303 and is, thus, an indication of the flow of contrast agent in the hepatic artery 12 over time. Similarly, the second time-intensity curve 304 corresponds with (*e.g.,* by fitting, averaging, *etc.*) the second contrast intensity information 305 and is, thus, an indication of the flow of contrast agent in the portal vein 10 over time.

Accordingly, the first time-intensity curve 302 correspond to the varying intensity of a first group of pixels of contrast flow in the first ROI drawn over the hepatic artery 12 in FIGURE 2 and is related to the flow of contrast agent in the hepatic artery over time. Similarly, the second time-intensity curve 304 corresponds to the varying intensity of a second group of pixels (i.e., those in the second ROI drawn over the portal vein 10 in FIGURE 3) and is related to the flow of contrast agent in the portal vein over time.

FIGURE 4 also illustrates a number of quantified parameters which may be obtained from the time-intensity curves (TICs). Indicated at 306 is the peak intensity of the TIC of the hepatic artery 12 and the time 310 from $t_1$ to $t_2$ is the time from the first arrival of contrast in the artery to the peak intensity, referred to as the wash-in time. The mean slope of the curve between times $t_1$ and $t_2$ may also be calculated if desired. Similarly, 308 indicates the peak intensity of the TIC of the portal vein 10 and the time interval 312 from $t_3$ to $t_4$ is the wash-in time from the first arrival of contrast in the vein to the peak intensity of contrast flow. Following the peak intensity points of both curves the curves decline as contrast agent washes out of the vessels. The durations and slopes of this portion of the curves may also be quantified if desired.

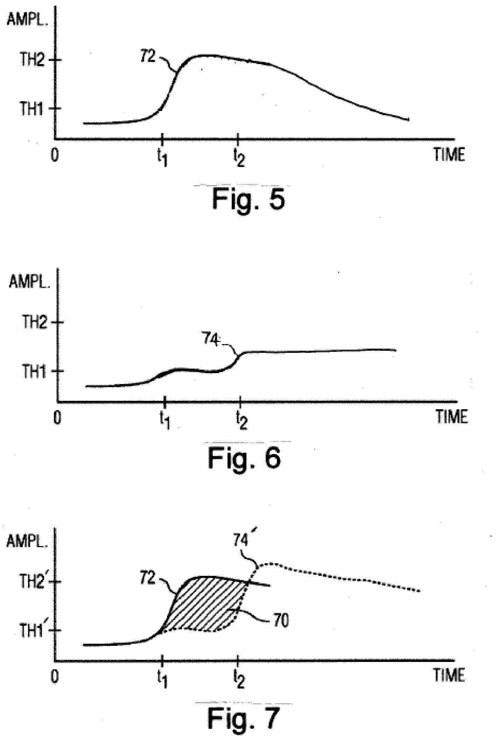

FIGURES 5 and 6 show examples of time-intensity curves for the hepatic artery 12 and portal vein 10, respectively. In FIGURE 5 the hepatic artery TIC 72 begins to rise from a level of threshold TH1 at a time $t_1$ to a peak threshold TH2 of peak contrast intensity. Thereafter the amount of contrast in the artery begins to decline, first slowly and then at a more rapid decline as the curve 72 illustrates. The portal vein TIC 74 in FIGURE 6 does not begin to rise until the later arrival of contrast in the portal vein at time $t_2$ and rises to a lesser peak intensity shortly thereafter, which is substantially sustained during the time of the curve 74 shown in the drawing.

The flow of contrast in the capillary structure of the liver parenchyma from these two sources of flow is a combination of both, as shown in FIGURE 7. The composite TIC in the parenchyma is curve 72 (from the hepatic arterial flow) and curve 74' where it exceeds the level of curve 72 after the arrival of the portal venous flow. Important in distinguishing between the two sources of flow is the shaded region 70 in FIGURE 7. This is a region demarcated by time interval $t_1 - t_2$, the time from the arrival of contrast from the hepatic artery to the later time of arrival of contrast from the portal vein. In FIGURE 4 this would be the time interval $t_1 - t_3$. This time is a temporal window during which the blood flow in the liver is only arterial blood flow from the hepatic vein; after time $t_2$ in FIGURE 7, the flow in the liver is a combination of flow from both sources.

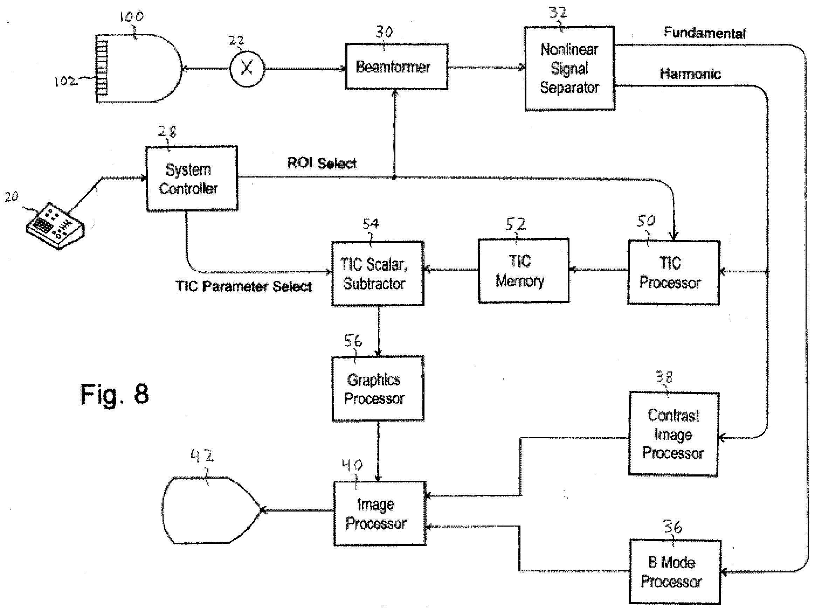

Referring now to FIGURE 8, an ultrasound system constructed in accordance with the principles of the present invention is shown in block diagram form. An ultrasonic probe 100 includes an array 102 of ultrasonic transducer elements that transmit and receive ultrasonic pulses. The array may be a one dimensional linear or curved array for two dimensional imaging, or may be a two dimensional matrix of transducer elements for electronic beam steering and focusing in two or three dimensions. The ultrasonic transducer elements in the array 102 transmit beams of ultrasonic energy by their timed actuation by a beamformer 30, and receive echoes returned in response to this transmission. A transmit/receive ("T/R") switch 22 is coupled to the ultrasonic transducers in the array 102 to selectively couple signals from the transducer elements to A/D converters in the beamformer 30 during the receive phase of operation. The times at which the transducer array is actuated to transmit signals may be synchronized to an internal system clock (not shown), or may be synchronized to a bodily function such as the heart cycle, for which a heart cycle waveform is conventionally provided by an ECG waveform sensor coupled to the ultrasound system. When the heartbeat is at the desired phase of its cycle as determined by the waveform provided by ECG sensor, the probe is commanded by a system controller to acquire an ultrasonic image.

Echoes from the transmitted ultrasonic energy are received by the transducers of the array 102, which generate echo signals that are coupled through the T/R switch 22 and digitized by the analog to digital converters when the system uses a digital beamformer. Analog beamformers may alternatively be used. The A/D converters sample the received echo signals at a sampling frequency controlled by a signal $f_s$ generated by a system controller 28. The desired sampling rate dictated by sampling theory is at least twice the highest frequency of the received passband, and might be on the order of 30-40 MHz. Sampling rates higher than the minimum requirement are also desirable. Control of the ultrasound system and of various control setting for imaging such as probe selection and ROI delineation is effected by user manipulation of the controls of a user control panel 20 which is coupled to and applies its control through the system controller 28.

The echo signal samples from the individual transducers of the array 14 are delayed and summed by the beamformer 30 to form coherent echo signals along scanline directions for an image. The digital coherent echo signals may then be filtered by a digital filter and undergo noise reduction as by spatial or frequency compounding. The digital filter can also shift the frequency band to a lower or baseband frequency range. The digital filter could be a filter of the type disclosed in U.S. Patent No. 5,833,613 (Averkiou et al.), for example. When phase information is needed as is the case for Doppler processing, quadrature (I and Q) demodulation may also be performed on the echo signals. In this implementation, the transmit frequency $f_o$ and the receiver frequency are individually controlled so that the beamformer 32 is free to receive a band of frequencies which is different from that of the transmitted band such as a harmonic frequency band around frequency $2f_o$.

The beamformed coherent echo signals are coupled to a nonlinear signal separator 32. The nonlinear signal separator 32 preferably separates harmonic frequency echoes returned from harmonic contrast agents by the pulse inversion technique, in which echo signals resulting from the transmission of multiple, differently phased (inverted) pulses to an image location are additively combined to cancel fundamental signal components and enhance harmonic components, thus producing echo signals in a harmonic band $2f_o$. The same echo signals are subtractively combined to produce echo signals in a fundamental frequency band $f_o$. A preferred pulse inversion technique is described in U.S. patent 6,186,950 (Averkiou et al.) and in U.S. patent 5,706,819 (Hwang et al.) for instance.

Harmonic echo signals from a contrast agent, such as microbubbles, are coupled to a contrast image processor 38. Contrast agents are often used to more clearly delineate blood vessels, or to perform perfusion studies of the microvasculature of tissue as described in US Pat. 6,692,438 (Skyba et al.) for example. In the implementation shown in FIGURE 8, echoes from a contrast agent are used to produce both a contrast image as shown in FIGURES 2 and 3, and time intensity curves from selected ROIs in the image field. The contrast agent processor produces a contrast image by amplitude (or envelope) detection of the harmonic frequency echoes from each point in the image field. One way to do this when the echoes are quadrature demodulated is to calculate the amplitude in the form of $(I^2+Q^2)^{1/2}$. These contrast intensity signals are mapped to the desired display format by scan conversion which converts samples from $R-\theta$ coordinates to Cartesian $(x,y)$ coordinates for display of a spatially defined image as shown in FIGURES 2 and 3.

The fundamental frequency echo signals are coupled to a B mode processor 36 which produces a standard B mode tissue image. The B mode processor performs in the same manner as the contrast image processor, but operates on fundamental frequency echoes. The echo signals are amplitude (envelope) detected and scan converted to produce a spatially delineated image of tissue such as the B mode images shown on the right side of FIGURES 2 and 3. The contrast and B mode images are coupled to an image processor 40 which does the image processing needed to display the images on an image display 42. This may include displaying both images at the same time, side-by-side, as shown in FIGURES 2 and 3. It may also comprise overlaying the contrast images over the B mode images so that the flow of blood containing contrast agent is shown in relation to the tissue structure of the vessels in which it is flowing.

In accordance with the principles of the present invention the harmonic echo signals are coupled to a TIC processor 50. The TIC processor processes echo signals from a contrast agent received over time from a region of interest (ROI) to produce a time-intensity curve such as those shown in FIGURES 4-7. As mentioned above, this processing involves acquiring echo signals over time from contrast agent washing into and out of the vessel(s) of a region of interest and fitting this echo data to a smooth curve. Since a contrast agent can be applied in a bolus injection, and can also be disrupted by relatively intense ultrasound which breaks or diffuses the microbubbles which are then allowed to reperfuse the vessels in the ROI, temporal characteristics of the arrival and departure of the contrast agent can be measured and used for diagnosis. A common measure is the time-intensity curve of the arrival and departure of the contrast agent as described in US Pat. 5,833,613 (Averkiou et al.) A time-intensity curve can be calculated for each point in an image of perfused tissue and one or more parameters of each curve for each image point can be displayed in grayscale shades or color-coding to form a parametric image of perfusion as described in US Pat. 6,692,438 (Skyba et al.) These parameters include the peak and the slope of the curves, each indicating a different characteristic of the tissue perfusion.

A time-intensity curve is generally computed by measuring the intensity of signals returned from the contrast agent as it flows into and out of blood vessels and the microvasculature of the tissue. These measurements of the rise and fall of the amount of contrast agent are then fitted to a curve such as that defined by the Gamma-variate curve model

$$A*(x-t_0)*\exp(-\rho*(x-t_0))+C,$$

where $A$ is the curve peak, $t_0$ is the time of initiation of the increase of contrast agent, $\rho$ is the slope of the rise of the curve, and $x$ is the instantaneous measurement of the amount of the contrast agent. These time and intensity representations provide an indication to a trained clinician of the manner in which the tissue is perfused.

In an implementation of the present invention a time-intensity curve is formed from the contrast wash-in and wash-out in an ROI located over the hepatic artery 12 as shown in FIGURE 2, and from the contrast wash-in and wash-out in an ROI located over the portal vein 10 as shown in FIGURE 3. This is done by selection of an ROI manually by the user by manipulating a control to move a cursor to the ROI in an image or by automated selection means, and providing the spatial location of the ROI by an "ROI Select" signal to the TIC processor. The ROI Select signal may also be provided to the beamformer to instruct the beamformer where to acquire the required echoes for time-intensity curve processing. The TIC from the hepatic artery will appear similar to that shown in FIGURE 5 (and as curve 302 in FIGURE 4), and the TIC from the portal vein will appear similar to that shown in FIGURE 6 (and as curve 304 in FIGURE 4). The resulting curves are stored as hepatic and portal reference curves in a TIC memory 52. Then a time-intensity curve is produced from the reperfusion of contrast in an ROI of the liver parenchyma. In accordance with the principles of the present invention, the flow into the parenchyma from the two sources of blood, the hepatic artery and the portal vein is then estimated. The reference curve acquired from the hepatic artery 12 is related to the TIC of the ROI which is a combination of the flow from both sources. Since the TIC from the hepatic artery is from contrast in pure blood flow, it will have a significantly greater intensity (amplitude) than the TIC from the liver ROI. The hepatic artery TIC (302,72) is therefore scaled in amplitude (and time, if needed) to match the amplitude of the liver TIC, principally using the portion of the hepatic artery TIC occurring during time window $t_1$-$t_2$ in FIGURES 5-7 when the flow in the liver is only from the hepatic artery. The thus-scaled hepatic artery TIC is then used as an estimate of the flow characteristic in the liver ROI resulting only from flow from the hepatic artery.

Since the combined flow in the liver ROI is linearly related to the flow of blood from both sources, the scaled hepatic artery TIC is then subtracted from TIC from the liver ROI, which, for instance, is the combination of curves 72 and 74' in FIGURE 7. This subtraction will result in a curve similar to curve 74 of FIGURE 6 but at the lower amplitude (compared to the portal vein curve) of the tissue perfusion curve from the liver ROI. The resulting curve is then used as an estimate of the flow characteristic in the liver ROI resulting only from flow from the portal vein.

One or more characteristics of these estimated curves are coupled to a graphics processor 56 which displays them to the clinician for diagnostic purposes. A simple display is to display the data of the two estimated curves of arterial and venous perfusion for a selected ROI on the display screen, which will appear similar to the curves shown in FIGURES 5 and 6. A more complete two-dimensional map for the liver in the image field may also be produced. For instance, estimated arterial and venous curve data maybe calculated for each point in the liver in an image, and a parameter of the curves shown qualitatively at each point in the liver as a parametric overlay over a B mode image. Using the Gamma-variate curve equation above, the peak of each curve A is selected and converted to a color value which varies with the magnitude of A. A B mode image may then be overlaid with a color map indicating the peak intensity A of arterial perfusion at each point in the liver, or overlaid with a color map indicating the peak intensity A of venous perfusion at each point in the liver image. Other parameters may also be calculated for each point in an image, color-coded, and displayed, such as a parametric map of wash-in time of arterial blood flow ($t_1$-$t_2$ in FIGURE 4), wash-in time of venous blood flow ($t_3$-$t_4$ in FIGURE 4), or the slope of the wash-in time ($\rho$) for either arterial for venous flow. Other parameters which may be displayed will be readily apparent to those skilled in the art.

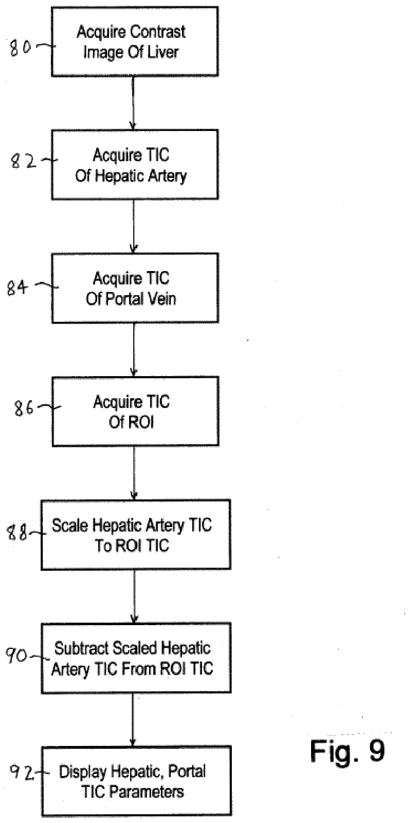

The steps of an exemplary method in accordance with the present invention is shown in FIGURE 9. In step 80 contrast images of the liver are acquired from a source of contrast-infused liver images, such as those shown in FIGURES 2 and 3. These maybe live (real time) images or an image loop previously acquired and retrieved from storage in memory. In step 82 a time-intensity curve of flow in the hepatic artery is acquired. This may be done by placing an ROI over the hepatic artery 12 in the images as shown in FIGURE 2, acquiring contrast echo data from the hepatic flow during wash-in and wash-out, and processing the data in the TIC processor 50 to produce an arterial reference TIC. In step 84 a time-intensity curve of flow in the portal vein is acquired. This may be done by placing an ROI over the portal vein 10 in the images as shown in FIGURE 3, acquiring contrast echo data from the venous flow during wash-in and wash-out, and processing the data in the TIC processor 50 to produce a venous reference TIC. In step 86 a time-intensity is acquired from the flow in a liver ROI. As mentioned above, only one liver ROI may be used, or time-intensity curves acquired over a portion or all of the liver parenchyma. In step 88 the larger amplitude arterial TIC is scaled to the size and duration of the liver ROI curve, using the period of initial wash-in when flow in the liver is due only to hepatic arterial flow. The scaled arterial TIC data is used as an estimated time-intensity curve of flow in the ROI due to blood from only the hepatic artery. In step 90 the scaled arterial TIC data is subtracted from the liver ROI curve to produce time-intensity curve data which is an estimate of the blood flow in the liver ROI due to only venous flow. In step 92 one or more parameters of the arterial and venous flow curves are displayed to the user, such as displaying the estimated arterial and venous curves of a single ROI in the liver, or a parametric map of time-intensity curve parameters for some or all of the liver on the display.

It should be noted that the ultrasound system which acquires contrast data from flow in the hepatic artery, portal vein, and liver and produces estimates of flow in the liver due to the individual sources of blood flow as shown in FIGURE 8 and in the method of FIGURE 9, and in particular the component structure of the ultrasound system of FIGURE 8, may be implemented in hardware, software or a combination thereof. The various embodiments and/or components of an ultrasound system, for example, the modules, or components and controllers therein, also may be implemented as part of one or more computers or microprocessors. The computer or processor may include a computing device, an input device, a display unit and an interface, for example, for accessing the Internet. The computer or processor may include a microprocessor. The microprocessor maybe connected to a communication bus, for example, to access a PACS system. The computer or processor may also include a memory. The memory devices such as the A-line memory 24 may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including the acquisition of contrast data and the calculation of time-intensity curves and parameters described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules, a program module within a larger program or a portion of a program module. For example, the equations calculated by the time-intensity data processors of FIGURE 8 may be executed by software modules calculating the equations. The software also may include modular programming in the form of object-oriented programming. The processing of input data by the processing machine maybe in response to operator commands, or in response to results of previous processing, or in response to a request made by another processing machine. In the imaging and time-intensity calculation portions of FIGURE 8, for instance, software instructions are conventionally employed to calculate the equations given above.

Furthermore, the limitations of the following claims are not written in means-plus-function format and are not intended to be interpreted based on 35 U.S.C. 112, sixth paragraph, unless and until such claim limitations expressly use the phrase "means for" followed by a statement of function devoid of further structure.

**Claims**

1. An ultrasonic diagnostic imaging system for diagnosing characteristics of liver perfusion comprising:

a source of liver images acquired in the presence of contrast agent flow;

a nonlinear signal separator, coupled to receive the liver images acquired in the presence of contrast agent flow, and configured to produce echo signal data from contrast agent flow;

a time-intensity curve processor, responsive to the echo signal data from contrast agent flow, which is configured to produce time-intensity curves of contrast agent flow in a hepatic artery, a portal vein, and in at least one region of interest (ROI) of a liver;

a time-intensity curve scalar and subtractor, configured to produce time-intensity curve data representative of arterial flow in the ROI from the time-intensity curves of contrast agent flow in the hepatic artery and in the ROI, wherein the time-intensity curve scalar and subtractor is further configured to produce time-intensity curve data representative of venous flow in the ROI from the time-intensity curve of contrast agent flow in the ROI and the time-intensity curve data representative of arterial flow in the ROI; and

a display configured to display one or more of the time-intensity curve data representative of arterial flow in the ROI and the time-intensity curve data representative of venous flow in the ROI.

2. The ultrasonic diagnostic imaging system of Claim 1, further comprising a user control configured to select an ROI in a liver image.

3. The ultrasonic diagnostic imaging system of Claim 2, wherein the user control is further configured to apply the spatial location of an ROI in a liver image to the time-intensity curve processor.

4. The ultrasonic diagnostic imaging system of Claim 3, wherein the time-intensity curve scalar and subtractor is further configured to produce the time-intensity curve data representative of arterial flow in the ROI by scaling the time-intensity curve of contrast agent flow in the hepatic artery to the time-intensity curve of contrast agent flow in the ROI.

5. The ultrasonic diagnostic imaging system of Claim 4, wherein the time-intensity curve scalar and subtractor is further configured to produce the time-intensity curve data representative of venous flow in the ROI by subtracting the time-intensity curve data representative of arterial flow in the ROI from the time-intensity curve of contrast agent flow in the ROI.

6. The ultrasonic diagnostic imaging system of Claim 1, wherein the display is further configured to display a time-intensity curve representative of arterial flow in the ROI and a time-intensity curve representative of venous flow in the ROI.

7. The ultrasonic diagnostic imaging system of Claim 1, wherein the display is further configured to display a parametric map of arterial flow or venous flow in a liver.

8. The ultrasonic diagnostic imaging system of Claim 7, wherein the display is further configured to display the parametric map overlaid on a B mode image of the liver.

9. A method for ultrasonically displaying liver perfusion parameters comprising:

acquiring contrast agent flow images of a liver;
producing a time-intensity curve of contrast agent flow in a hepatic artery location of the images;
producing a time-intensity curve of contrast agent flow in a portal vein location of the images;
producing a time-intensity curve of contrast agent flow in a region of interest (ROI) of the liver;
producing time-intensity curve data representative of arterial flow in the ROI based on the time-intensity curve of contrast agent flow in the ROI of the liver and the time-intensity curve of contrast agent flow in a hepatic artery location;
producing time-intensity curve data representative of venous flow in the ROI based on the time-intensity curve data representative of arterial flow in the ROI and the time-intensity curve of contrast agent flow in the ROI; and
displaying one or more of the time-intensity curve data representative of arterial flow in the ROI and the time-intensity curve data representative of venous flow in the ROI.

10. The method of Claim 9, further comprising selecting a region of interest in an image of the liver.

11. The method of Claim 10, wherein producing time-intensity curve data representative of arterial flow in the ROI further comprises scaling the time-intensity curve of contrast agent flow in a hepatic artery location to match the time-intensity curve of contrast agent flow in the ROI.

12. The method of Claim 11, wherein scaling further comprises matching an initial rise of the time-intensity curve of contrast agent flow in the hepatic artery location to an initial rise of the time-intensity curve of contrast agent flow in the ROI.

13. The method of Claim 11, wherein producing time-intensity curve data representative of venous flow in the ROI further comprises subtracting the time-intensity curve data representative of arterial flow in the ROI from the time-intensity curve of contrast agent flow in the ROI.

14. The method of Claim 9 wherein displaying further comprises displaying a parametric map of one or more of the time-intensity curve data representative of arterial flow in the liver and the time-intensity curve data representative of venous flow in the liver.

15. A computer program product comprising instructions which, when the computer program product is executed by a computer, cause the computer to carry out the method of any of Claims 9-14.

**Fig. 1**

**Fig. 2**

**Fig. 3**

Fig. 4

Fig. 5

Fig. 6

Fig. 7

Fig. 8

80 — Acquire Contrast Image Of Liver

82 — Acquire TIC Of Hepatic Artery

84 — Acquire TIC Of Portal Vein

86 — Acquire TIC Of ROI

88 — Scale Hepatic Artery TIC To ROI TIC

90 — Subtract Scaled Hepatic Artery TIC From ROI TIC

92 — Display Hepatic, Portal TIC Parameters

Fig. 9

Europäisches
Patentamt

European
Patent Office

Office européen
des brevets

**EUROPEAN SEARCH REPORT**

Application Number

EP 17 16 1867

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LUECK G J ET AL: "Hepatic Perfusion Imaging Using Factor Analysis of Contrast Enhanced Ultrasound", IEEE TRANSACTIONS ON MEDICAL IMAGING, IEEE SERVICE CENTER, PISCATAWAY, NJ, US, vol. 27, no. 10, 1 October 2008 (2008-10-01), pages 1449-1457, XP011226135, ISSN: 0278-0062, DOI: 10.1109/TMI.2008.922695 * the whole document * | 1-15 | INV. A61B8/06 A61B8/08 |
| A | WO 2011/041244 A1 (KONINKL PHILIPS ELECTRONICS NV [NL]; PHILIPS CORP [US]; GAUTHIER THOMA) 7 April 2011 (2011-04-07) * page 4, line 17 - page 7, line 9 * * page 17, line 1 - page 22, line 7; figures * | 1-15 | |
| A | THOMAS P. GAUTHIER ET AL: "Reproducibility of Quantitative Assessment of Altered Hepatic Hemodynamics With Dynamic Contrast-Enhanced Ultrasound", JOURNAL OF ULTRASOUND IN MEDICINE., vol. 31, no. 9, 18 January 2012 (2012-01-18), pages 1413-1420, XP055408157, United States ISSN: 0278-4297, DOI: 10.7863/jum.2012.31.9.1413 * the whole document * | 1-15 | TECHNICAL FIELDS SEARCHED (IPC) A61B G01S |

-/--

The present search report has been drawn up for all claims

| Place of search | Date of completion of the search | Examiner |
|---|---|---|
| Munich | 20 September 2017 | Küster, Gunilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 17 16 1867

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2009/093211 A1 (AVERKIOU MICHALAKIS [CY]; LAMPASKIS MARIOS [CY]; KYRIAKOPOULOU KONSTAN) 30 July 2009 (2009-07-30) <br> * page 3, line 5 - page 4, line 11 * <br> * page 11, line 11 - page 13, line 10; figures * <br> ----- | 1-15 | |
| A | DONG LIU ET AL: "Hepatic Perfusion Parameters of Contrast-Enhanced Ultrasonography Correlate With the Severity of Chronic Liver Disease", ULTRASOUND IN MEDICINE AND BIOLOGY., vol. 40, no. 11, 1 November 2014 (2014-11-01), pages 2556-2563, XP055408158, US ISSN: 0301-5629, DOI: 10.1016/j.ultrasmedbio.2014.05.011 * the whole document * <br> ----- | 1-15 | |

**TECHNICAL FIELDS SEARCHED (IPC)**

The present search report has been drawn up for all claims

| Place of search | Date of completion of the search | Examiner |
|---|---|---|
| Munich | 20 September 2017 | Küster, Gunilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**ANNEX TO THE EUROPEAN SEARCH REPORT
ON EUROPEAN PATENT APPLICATION NO.**

EP 17 16 1867

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2017

| Patent document cited in search report | | | Publication date | Patent family member(s) | | | Publication date |
|---|---|---|---|---|---|---|---|
| WO 2011041244 | A1 | | 07-04-2011 | CN | 102573647 | A | 11-07-2012 |
| | | | | US | 2012253190 | A1 | 04-10-2012 |
| | | | | WO | 2011041244 | A1 | 07-04-2011 |
| WO 2009093211 | A1 | | 30-07-2009 | AT | 523146 | T | 15-09-2011 |
| | | | | BR | PI0906934 | A2 | 28-07-2015 |
| | | | | CN | 101951839 | A | 19-01-2011 |
| | | | | CN | 101969857 | A | 09-02-2011 |
| | | | | EP | 2234544 | A1 | 06-10-2010 |
| | | | | EP | 2237725 | A1 | 13-10-2010 |
| | | | | JP | 2011509789 | A | 31-03-2011 |
| | | | | JP | 2011509790 | A | 31-03-2011 |
| | | | | RU | 2010134892 | A | 27-02-2012 |
| | | | | RU | 2010134895 | A | 27-02-2012 |
| | | | | US | 2010298706 | A1 | 25-11-2010 |
| | | | | US | 2010298710 | A1 | 25-11-2010 |
| | | | | WO | 2009093211 | A1 | 30-07-2009 |
| | | | | WO | 2009093212 | A1 | 30-07-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5833613 A, Averkiou
- US 6186950 B, Averkiou
- US 5706819 A, Hwang
- US 6692438 B, Skyba

**Non-patent literature cited in the description**

- **M. AVERKIOU et al.** Ultrasound Contrast Imaging Research. *Ultrasound Quarterly,* 2003, vol. 19 (1), 27